# EUROPÄISCHE PATENTSCHRIFT

(11) **EP 1 351 921 B1**
(45) Veröffentlichungstag und Bekanntmachung des Hinweises auf die Patenterteilung: **01.12.2004**
(21) Anmeldenummer: 01983476.1
(22) Anmeldetag: 10.09.2001
(51) Int. Cl.: C07C 211/51, A61K 7/13

(54) **NEUE 1,4-DIAMINO-2-ALKENYL-BENZOL-DERIVATE UND DIESE VERBINDUNGEN ENTHALTENDE FÄRBEMITTEL**
NOVEL 1,4-DIAMINO-2-ALKENYL-BENZENE DERIVATIVES AND COLORANTS CONTAINING SAID COMPOUNDS
NOUVEAUX DERIVES DE 1,4-DIAMINO-2-ALCENYL-BENZOL ET COLORANTS CONTENANT CES COMPOSES

(30) Priorität: 18.01.2001 DE 10102085
(43) Veröffentlichungstag der Anmeldung: 15.10.2003
(73) Patentinhaber: Wella Aktiengesellschaft, 64274 Darmstadt (DE)
(72) Erfinder: CHASSOT, Laurent, CH-1724 Praroman (CH); BRAUN, Hans-Jürgen, CH-3182 Überstorf (CH)
(86) Internationale Anmeldenummer: PCT/EP2001/010408
(87) Internationale Veröffentlichungsnummer: WO 2002/057214

(56) Entgegenhaltungen:
- EP-A- 0 634 163
- EP-A- 0 819 424

## Beschreibung

Die Erfindung betrifft neue 1,4-Diamino-2-alkenyl-benzol-Derivate sowie diese Verbindungen enthaltende Mittel zum Färben von Keratinfasem.

Auf dem Gebiet der Färbung von Keratinfasem, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol 1,4-Diaminobenzol und 4,5-Diaminopyrazol eingesetzt, während als Kupplersubstanzen beispielsweise Resorcin, 4-Chlorresorcin, 1-Naphthol, 3-Aminophenol, m-Phenylendiamin, 2-Amino-4-(2'-hydroxyethyl)amino-anisol, 1,3-Diamino-4-(2'-hydroxyethoxy)benzol und 2,4-Diamino-5-fluor-toluol zu nennen

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann.

In der EP-PS 0 819 424 sowie der EP-OS 0 634 163 werden Oxidationshaarfärbemittel beschrieben, die als Entwicklersubstanz unter andere 2-Alkyl-p-phenylendiamine oder deren Salze enthalten.

Es bestand jedoch weiterhin ein Bedürfnis nach neuen Entwicklersubstanzen, welche die an Oxidationsfarbstoffvorstufen gestellten Anforderungen in besonderem Masse erfüllen.

Hierzu wurde nun überraschenderweise gefunden, daß neue p-Diaminobenzol-Derivate gemäß der allgemeinen Formel (I) die an Entwicklersubstanzen gestellten Anforderungen in besonders hohem Masse erfüllen. So werden unter Verwendung dieser Entwicklersubstanzen mit den meisten bekannten Kupplersubstanzen farbintensive Farbnuancen erhalten, die außerordentlich lichtecht und waschecht sind.

Gegenstand der vorliegende Erfindung sind daher 1,4-Diamino-2-alkenylbenzol-Derivate der allgemeinen Formel (I) oder deren physiologisch verträgliche, wasserlösliche Salze, 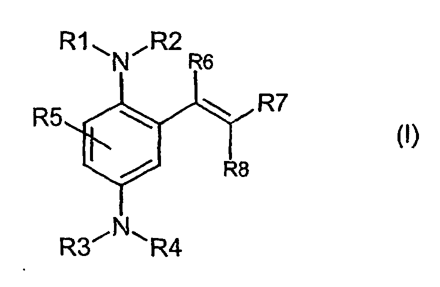 worin
**R1,R2,R3 und R4** unabhängig voneinander Wasserstoff, eine C₁-C₆-Alkylgruppe, eine C₂-C₄-Hydroxyalkylgruppe, eine C₃-C₄- Dihydroxyalkylgruppe oder eine C₁-C₄-Alkoxy-(C₁-C₂)-alkylgruppe darstellen oder R1und R2 beziehungsweise R3 und R4 einen viergliedrigen bis achtgliedrigen aliphatischen Ring bilden, wobei mindestens zwei der Reste R1 bis R4 Wasserstoff darstellen;
**R5** gleich Wasserstoff, einem Halogenatom (F, Cl, Br, J), einer C₁-C₄-Alkylgruppe, einer C₁-C₄ -Hydroxyalkylgruppe oder einer C₁-C₄-Alkoxygruppe ist;
**R6, R7 und R8** unabhängig voneinander gleich Wasserstoff, einem Halogenatom (F, Cl, Br, J), einer Cyanogruppe, einer C₁-C₄ -Alkoxygruppe, einer C₁-C₆ Alkylgruppe, einer -C(O)H-Gruppe, einer -Si(CH₃)₃-Gruppe, einer C₁-C₄-Hydroxyalkylgruppe, einer C₃-C₄-Dihydroxyalkylgruppe, einer -CH2-Si(CH₃)₃-Gruppe, oder einem Rest der Formel (II) sind, 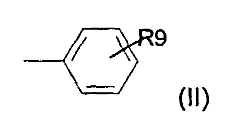 **R9** gleich Wasserstoff, einer Aminogruppe, einem Halogenatom F, Cl, Br, J), einer Nitrogruppe oder einer Hydroxygruppe ist.

Als geeignete Verbindungen der Formel (I) können beispielweise die folgenden Verbindungen genannt werden:
1,4-Diamino-2-styryl-benzol; 1,4-Diamino-2-propenyl-benzol; 1,4-Diamino-2-(1-methyl-propenyl)-benzol; 1,4-Diamino-2-(2-methyl-propenyl)-benzol; 1,4-Diamino-2-(1,2-Dimethyl-propenyl)-benzol; 1 Amino-4-methylamino-2-styryl-benzol; 2-(2,5-Diamino-phenyl)-prop-2-en-1-ol; 3-(2,5-Diaminophenyl)-prop-2-en-1-ol; 1 Amino-4-methylamino-2-(1-methyl-propenyl)-benzol; 1 Amino-4-methylamino-2-(2-methyl-propenyl)-benzenol; 1-Amino-4-methylamino-2-(1,2-dimethyl-propenyl)-benzol; 1-Amino-4-(2-hydroxyethyl)amino)-2-styryl-benzol; 1-Amino-4-(2-hydroxyethyl)amino)-2-propenyl-benzol, 1-Amino-4-(2-hydroxyethyl)amino)-2-(1-methylpropenyl)-benzol; 1-Amino-4-(2-hydroxyethyl)amino)-2-(2-methylpropenyl)-benzenol; 1-Amino-4-(2-hydroxyethyl)amino)-2-(1,2-dimethylpropenyl)-benzol; 1-Amino-4-di(2-hydroxyethyl)amino-2-styryl-benzol; 1-Amino-4-di(2-hydroxyethyl)amino -2-propenyl-benzol; 1-Amino-4-di(2-hydroxyethyl)amino-2-(1-methyl-propenyl)-benzol; 1-Amino-4-di(2-hydroxyethyl)amino-2-(2-methyl-propenyl)-benzenol und 1-Amino-4-di(2-hydroxyethyl)amino-2-(1,2-dimethyl-propenyl)-benzol.

Bevorzugt sind Verbindungen der Formel (I), bei denen (i) eine oder mehrere der Restgruppen R5, R6, R7 und R8 gleich Wasserstoff sind und/oder (ii) R1, R2, R3 und R4 gleichzeitig Wasserstoff bedeuten und/oder (iii) eine oder mehrere der Restgruppen R6, R7 und R8 gleich C₁-C₄-Alkyl oder C₁-C₄-Hydroxyalkyl sind.

Insbesondere sind die folgenden Verbindungen zu nennen:
1,4-Diamino-2-styryl-benzol; 1,4-Diamino-2-propenyl-benzol; 1,4-Diamino-2-(1-methyl-propenyl)-benzol; 1,4-Diamino-2-(2-methyl-propenyl)-benzenol; 1,4-Diamino-2-(1,2-dirnethyl-propenyl)-benzol; 1,4-Diamino-2-propenyl-benzol; 2-(2,5-Diamino-phenyl)-prop-2-en-1-ol; 3-(2,5-Diaminophenyl)-prop-2-en-1-ol und 3-(2,5-Diamino-phenyl)-prop-2-en-1-ol.

Die 1,4-Diamino-2-alkenyl-benzol-Derivate der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die Herstellung der erfindungsgemäßen 1,4-Diamino-2-alkenyl-benzol-Derivate der Formel (I) kann unter Verwendung von bekannten Syntheseverfahren erfolgen. Die Synthese der erfindungsgemäßen Verbindungen kann beispielsweise wie folgt durchgeführt werden:
Entweder a) durch eine Tetrakis(triphenylphospin)palladium(0) katalysierte Kupplung eines substituierten Benzols der Formel (III) 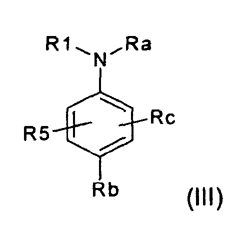 mit einer Verbindung der Formel (IV) 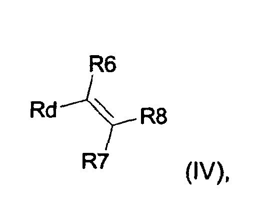 worin
   Ra die Bedeutung einer Schutzgruppe, wie sie zum Beispiel in dem Kapitel "Protective Groups" in Organic Synthesis, Kapitel 7, Wiley Interscience, 1991 beschrieben wird,
   Rb die Bedeutung NR1Ra oder NO₂ hat,
   Rc die Bedeutung Halogen und Rd die Bedeutung B(OH)₂ beziehungsweise Rc die Bedeutung B(OH)₂ und Rd die Bedeutung Halogen hat, und
   R1, R5, R6, R7 und R8 die in Formel (I) genannte Bedeutung haben, und
   anschließende Abspaltung der Schutzgruppe oder Abspaltung der Schutzgruppe und Reduktion der Nitrogruppe;
oder b) durch eine Tetrakis(triphenylphospin)palldium(0) katalysierte Kupplung eines substituierten Benzols der Formel (V) 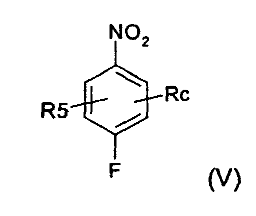 mit der vorstehend genannten Verbindung der Formel (IV) (wobei die Reste Rc, Rd, R5, R6, R7 und R8 die für die Formeln (I) bis (IV) vorstehend genannte Bedeutung haben;
   anschliessende Substitution des so erhaltenen substituierten Benzols der Formel (VI) 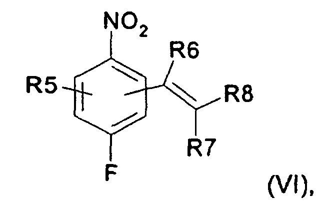 mit einem Amin der Formel HNR1R2,
   worin R1, R2 die in Formel (I) genannte Bedeutung haben, und
   abschliessende Reduktion der Nitrogruppe.

Die erfindungsgemäßen 1,4-Diamino-2-alkenyl-benzol-Derivate der Formel (I) sind in Wasser gut löslich und ermöglichen Färbungen mit hoher Farbintensität und ausgezeichneter Farbechtheit, insbesondere was die Lichtechtheit, Waschechtheit und Reibeechtheit anbetrifft. Die Verbindungen der Formen (I) weisen weiterhin eine ausgezeichnete Lagerstabilität, insbesondere als Bestandteil der nachfolgend beschriebenen Färbemittel, auf.

Ein weiterer Gegenstand der vorliegenden Erfindung sind daher Mittel zum oxidativen Färben von Keratinfasem, wie zum Beispiel Haaren, Pelzen, Federn oder Wolle, insbesondere menschlichen Haaren, auf der Basis einer Entwicklersubstanz-Kupplersubstanz-Kombination, welche als Entwicklersubstanz mindestens ein 1,4-Diamino-2-alkenyl-benzol-Derivat der Formel (I) enthalten.

Das 1,4-Diamino-2-alkenyl-benzol-Derivat der Formel (I) ist in dem erfindungsgemäßen Färbemittel in einer Menge von etwa 0,005 bis 20 Gewichtsprozent enthalten, wobei eine Menge von etwa 0,01 bis 5,0 Gewichtsprozent und insbesondere 0,1 bis 2,5 Gewichtsprozent bevorzugt ist.

Als Kupplersubstanzen kommen vorzugsweise N-(3-Dimethylaminophenyl)-harnstoff, 2,6-Diamino-pyridin, 2-Amino-4-[(2-hydroxyethyl)-amino]-anisol, 2,4-Diamino-1-fluor-5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlorphenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion in Betracht.

Obwohl die vorteilhaften Eigenschaften der hier beschriebenen 1,4-Diamino-2-alkenyl-benzol-Derivate der Formel (I) es nahelegen, diese als alleinige Entwicklersubstanz zu verwenden, ist es selbstverständlich auch möglich, die 1,4-Diamino-2-alkenyl-benzol-Derivate der Formel (I) gemeinsam mit bekannten Entwicklersubstanzen, wie zum Beispiel 1,4-Diaminobenzol, 2,5-Diaminotoluol, 2,5-Diaminophenylethylalkohol, 4-Aminophenol und seinen Derivaten, beispielsweise 4-Amino-3-methylphenol, 4,5-Diamino-1-(2-hydroxyethyl)-pyrazol, 4,5-Diamino-1-benzylpyrazol, 4,5-Diamino-1-(4-methyl-benzyl)-pyrazol oder Tetraaminopyrimidinen, einzusetzen.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6,0 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Uberschuß oder Unterschuß vorhanden sind.

Weiterhin kann das erfindungsgemäße Färbemittel zusätzlich andere Farbkomponenten, beispielsweise 6-Amino-2-methylphenol und 2-Amino-5-methylphenol, sowie ferner übliche direktziehende Farbstoffe, zum Beispiel Triphenylmethanfarbstoffe wie 4-[(4'-aminophenyl)-(4'-imino-2",5"cyclohexadien-1 "-yliden)-methyl]-2-methylaminobenzol-monohydrochlorid (C.l. 42 510) und 4-[(4'amino-3'-methyl-phenyl)-(4"-imino-3"-methyl-2",5"cyclohexadien-1"-yliden)-methyl]-2-methyl-aminobenzol monohydrochlorid (C.I. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl)-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5hydroxynaphthalin-1-sulfonsäure-Natriumsalz (C.I. 14 805) und Dispersionsfarbstoffe wie beispielsweise 1,4-Diaminoanthrachinon und 1,4,5,8-Tetraaminoantrachinon, enthalten. Die Färbemittel können diese Farbkomponenten in einer Menge von etwa 0,1 bis 4,0 Gewichtsprozent enthalten.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.
Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Färbemittel schwach sauer, neutral oder alkalisch reagieren. Insbesondere weist es einen pH-Wert von 6,5 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die erfindungsgemäßen Färbemittel mit einem Gehalt an 1,4-Diamino-2-alkenyl-benzol-Derivaten der Formel (I) als Entwicklersubstanz ermöglichen Haarfärbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Haarfärbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus. Die sehr guten färberischen Eigenschaften der Färbemittel gemäß der vorliegenden Anmeldung zeigen sich weiterhin darin, daß diese Mittel eine Anfärbung von ergrauten, chemisch nicht vorgeschädigten Keratinfasern, insbesondere menschlichen Haaren, problemlos und mit guter Deckkraft ermöglichen.

Die nachfolgenden Beispiele sollen den Gegenstand der Erfindung näher erläutern, ohne ihn darauf zu beschränken.

### Beispiele

### Beispiele 1: Synthese von 2,5-Diamino-1-(ethenyl)-benzolen (Allgemeine Synthesevorschrift)

### A. Synthese von N,N'-Bis(tert-Butoxycarbonyl)-2,5-diamino-1-phenylborsäure

Die N,N'-Bis(tert-Butoxycarbonyl)-2,5-diamino-1-phenylborsäure wird durch Umsetzung von N,N'-Bis(tert-Butoxycarbonyl)-2,5-diamino-1-brombenzol mit tert-Butyllithium und Trimethylborate gemäß der Vorschrift von J. M. Tour und J. J. S: Lamba in J. Am. Chem. Soc. 1994, 116, Seite 11723 dargestellt.

### B. Synthese von 2,5-Diamino-1-(vinyl)-benzolen

0,035 g (0,0001 mol) 2,5-tert.-Butyloxycarbonylamino-1-phenylborsäure aus Stufe A und 0,00015 mol des entsprechenden Bromderivates werden unter Argon in 10 ml 1,2-Dimethoxyethan gelöst. Anschließend werden 0,005 g Tetrakis-(triphenylphosphin)-palladium (0,000005 mol) und 0,13 ml einer 2normalen Kaliumcarbonatlösung zugegeben und die Reaktionsmischung auf 80 °C erwärmt. Nach Beendigung der Reaktion wird die Reaktionsmischung in 10 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol auf 50 °C erwärmt. Anschliessend werden zur Herstellung des Hydrochlorides 1,5 ml einer 2,9 molaren ethanolische Salzsäurelösung zugetropft. Der Niederschlag wird abfiltriert, zweimal mit 1 ml Ethanol gewaschen und sodann getrocknet.
**a.** 1,4-Diamino-2-propenyl-benzol Hydrochlorid
   Verwendetes Bromderivat: 1-Propenylbromid
   Ausbeute: 0,015 g ( 94 % der Theorie)
   Masspektrum: MH⁺ 149 (100)
**b.** 1,4-Diamino-2-(1-methyl-propenyl)-benzol Hydrochlorid
   Verwendetes Bromderivat: 1-Brom-1-methyl-propen
   Ausbeute: 0,015 g ( 95 % der Theorie)
   Masspektrum: MH⁺ 163 (100)
**c.** 1,4-Diamino-2-(2-methyl-propenyl)-benzol Hydrochloride
   Verwendetes Bromderivat: 1-Brom-2-methyl-1-propen
   Ausbeute: 0,012 g (90 % der Theorie)
   Masspektrum: MH⁺ 163 (100)
**d.** 1,4-Diamino-2-(1,2-dimethyl-propenyl)-benzol Hydrochloride
   Verwendetes Bromderivat: 1-Brom-1,2-dimethyl-1-propen
   Ausbeute: 0,012 g ( 80 % der Theorie)
   Masspektrum: MH⁺ 176 (100)
**e.** 1,4-Diamino-2-styryl-benzol Hydrochlorid
   Verwendetes Bromderivat: 1-Brom-2-phenyl-ethylen
   Ausbeute: 0,025 g ( 90 % der Theorie)
   Masspektrum: M⁺ 210 (10)

### Beispiele 2 bis 6: Haarfärbemittel

Es werden Haarfärbelösungen der folgenden Zusammensetzung hergestellt:

| | |
|---|---|
| 1,25 mmol | Entwicklersubstanz der Formel (I) gemäß Tabelle 1 |
| 1,25 mmol | Kupplersubstanz gemäß Tabelle 1 |
| 1,0 g | Kaliumoleat (8prozentige wässrige Lösung) |
| 1,0 g | Ammoniak (22prozentige wässrige Lösung) |
| 1,0 g | Ethanol |
| 0,3 g | Ascorbinsäure |
| ad 100,0 g | Wasser |

50 g der vorstehenden Färbelösung werden unmittelbar vor der Anwendung mit 50 g einer 6prozentigen wässrigen Wasserstoffperoxidlösung vermischt. Anschliessend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefasst.

**Tabelle 1:**

| **Beispiel Nr.** | **Entwicklersubstanz Der Formel (I)** | **Kupplersubstanz** | | | |
|---|---|---|---|---|---|
| | | **I.** **1,3-Di- hydroxy- benzol** | **II.** **1,3-Diamino-4- (2-hydroxy- ethoxy)- benzol-sulfat** | **III.** **5-Amino-2- methyl- phenol** | **IV.** **1-Naphtol** |
| **2.** | Gemäß Beispiel **1a** | dunkel-blond | dunkelblau | purpur | blau |
| **3.** | gemäß Beispiel **1b** | dunkel-blond | dunkelblau | purpur | blau |
| **4.** | gemäß Beispiel **1c** | dunkel-blond | blau-grau | purpur | blau |
| **5.** | gemäß Beispiel **1d** | blond | blau-grau | purpur | blau |
| **6.** | gemäß Beispiel **1e** | blond | blau | purpur | blau |

### Beispiele 7 bis 26: Haarfärbemittel

Es werden Haarfärbelösungen der folgenden Zusammensetzung hergestellt:

| | |
|---|---|
| X g | 1,4-Diamino-2-alkenyl-benzol-Derivat der Formel (I) (Entwicklersubstanz **E1** oder **E2** gemäß Tabelle 2) |
| U g | Entwicklersubstanz **E8** bis **E15** gemäß Tabelle 2 |
| Y g | Kupplersubstanz **K11** bis **K36** gemäß Tabelle 4 |
| Z g | direktziehender Farbstoff **D1** bis **D3** gemäß Tabelle 3 |
| 10,0 g | Kaliumoleat (8prozentige wässrige Lösung) |
| 10,0 g | Ammoniak (22prozentige wässrige Lösung) |
| 10,0 g | Ethanol |
| 0,3 g | Ascorbinsäure |
| ad 100,0 g | Wasser |

30 g der vorstehenden Färbelösung werden unmittelbar vor der Anwendung mit 30 g einer 6prozentigen wässsrigen Wasserstoffperoxidlösung vermischt. Anschliessend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die Färbeergebnisse sind in Tabelle 5 zusammengefasst.

### Beispiele 27 bis 38: Haarfärbemittel

Es werden cremeförmige Farbträgermassen der folgenden Zusammensetzung hergestellt:

| | |
|---|---|
| X g | 1,4-Diamino-2-alkenyl-benzol-Derivat der Formel (I) (Entwicklersubstanz **E1** oder **E2** gemäß Tabelle 2) |
| U g | Entwicklersubstanz **E8** bis **E15** gemäß Tabelle 2 |
| Y g | Kupplersubstanz **K11** bis **K36** gemäss Tabelle 4 |
| Z g | direktziehender Farbstoff **D2** gemäss Tabelle 3 |
| 15,0 g | Cetylalkohol |
| 0,3 g | Ascorbinsäure |
| 3,5 g | Natriumlaurylalkoholdiglycolethersulfat, 28prozentige wässrige Lösung |
| 3,0 g | Ammoniak, 22prozentige wässrige Lösung |
| 0,3 g | Natriumsulfit, wasserfrei |
| ad 100 g | Wasser |

30 g der vorstehenden Färbecreme werden unmittelbar vor der Anwendung mit 30 g einer 6prozentigen Wasserstoffperoxidlösung vermischt. Anschliessend wird das Gemisch auf das Haar aufgetragen. Nach einer Einwirkzeit von 30 Minuten bei 40 °C wird das Haar mit Wasser gespült, mit einem handelsüblichen Shampoo gewaschen und getrocknet. Die Färbeergebnisse sind in Tabelle 6 zusammengefasst.

Alle in der vorliegenden Anmeldung enthaltenen Prozentangaben stellen soweit nicht anders angegeben Gewichtsprozente dar.

**Tabelle 2:**

| **Entwicklersubstanzen** | |
|---|---|
| **E1** | 1,4-Diamino-2-propenyl-benzol Hydrochlorid |
| **E2** | 1,4-Diamino-2-(1,2-dimethyl-propenyl)-benzol Hydrochlorid |
| | |
| **E8** | 1,4-Diaminobenzol |
| **E9** | 2,5-Diamino-phenylethanol-sulfat |
| **E10** | 3-Methyl-4-amino-phenol |
| **E11** | 4-Amino-2-aminomethyl-phenol-dihydrochlorid |
| **E12** | 4-Amino-phenol |
| **E13** | N,N-Bis(2'-hydroxyethyl)-p-phenylendiamin-sulfat |
| **E14** | 4,5-Diamino-1-(2'-hydroxyethyl)-pyrazol-sulfat |
| **E15** | 2,5-Diaminotoluol-sulfat |

**Tabelle 3:**

| **Direktziehende Farbstoffe** | |
|---|---|
| **D1** | 2,6-Diamino-3-((pyridin-3-yl)azo)pyridin |
| **D2** | 6-Chlor-2-ethylamino-4-nitro-phenol |
| **D3** | 2-Amino-6-chlor-4-nitro-phenol |

**Tabelle 4:**

| **Kupplersubstanzen** | |
|---|---|
| **K11** | 1,3-Diaminobenzol |
| **K12** | 2-Amino-4-(2'-hydroxyethyl)amino-anisol-sulfat |
| **K13** | 1,3-Diamino-4-(2'-hydroxyethoxy)benzol-sulfat |
| **K14** | 2,4-Diamino-5-fluor-toluol-sulfat |
| **K15** | 3-Amino-2-methylamino-6-methoxy-pyridin |
| **K16** | 3,5-Diamino-2,6-dimethoxy-pyridin-dihydrochlorid |
| **K17** | 2,4-Diamino-5-ethoxy-toluol-sulfat |
| **K18** | N-(3-Dimethylamino)phenylhamstoff |
| **K19** | 1,3-Bis(2,4-Diaminophenoxy)propan-tetrahydrochlorid |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K26** | 1-Acetoxy-2-methyl-naphthalin |
| **K31** | 1,3-Dihydroxy-benzol |
| **K32** | 2-Methyl-1,3-dihydroxy-benzol |
| **K33** | 1-Chlor-2,4-dihydroxy-benzol |
| **K34** | 4-(2'-Hydroxyethyl)amino-1,2-methylendioxybenzol-hydrochlorid |
| **K35** | 3,4-Methylendioxy-phenol |
| **K36** | 2-Amino-5-methyl-phenol |

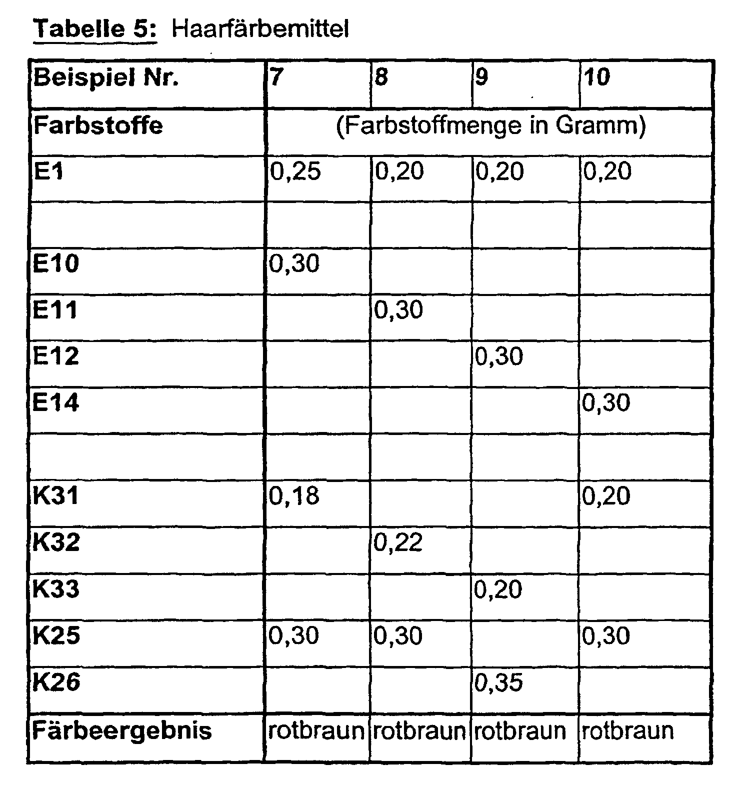

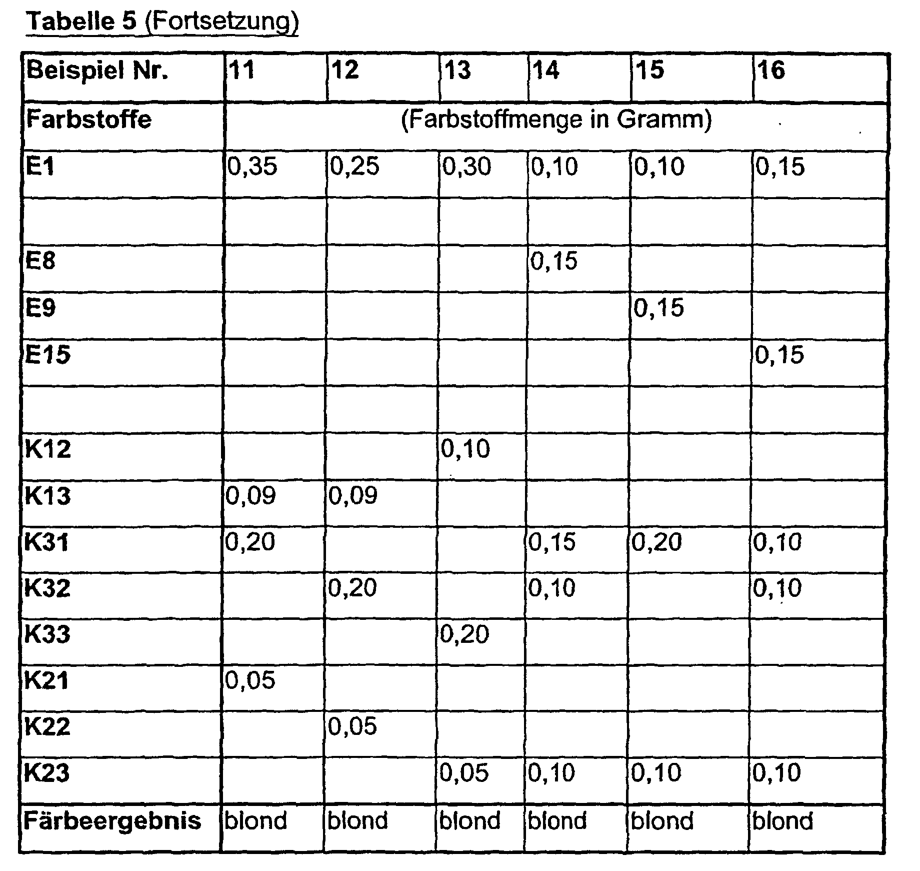

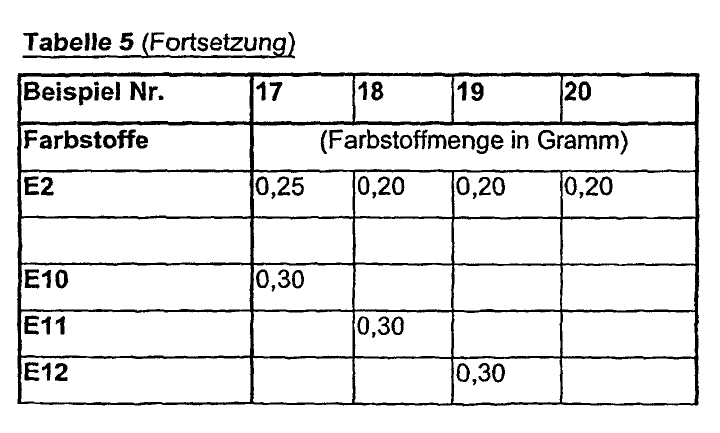

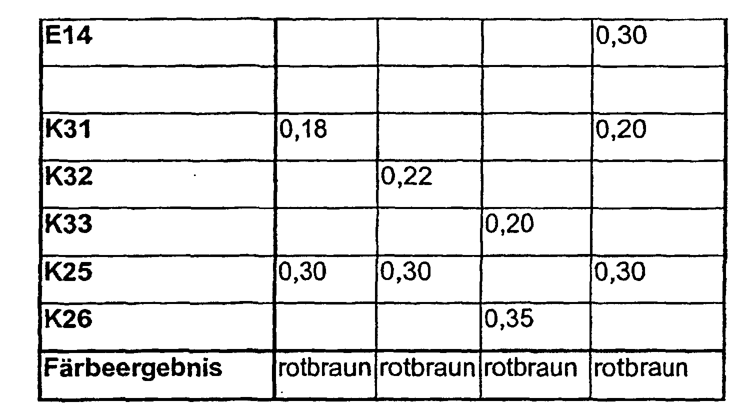

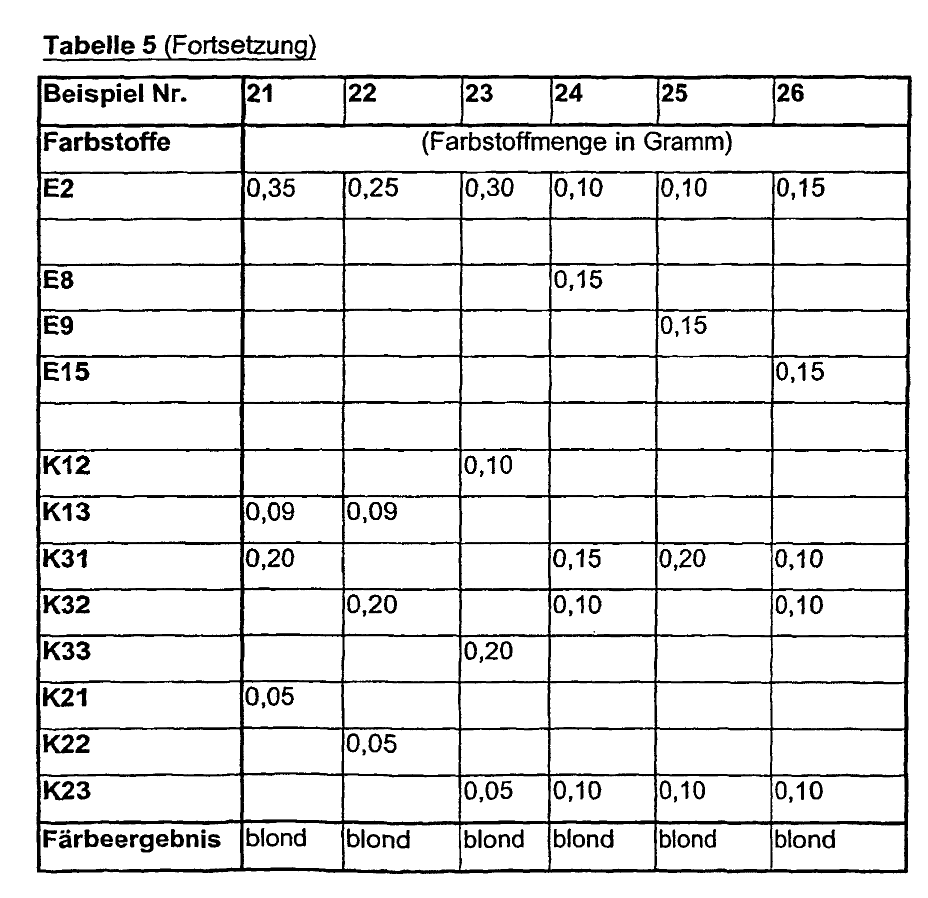

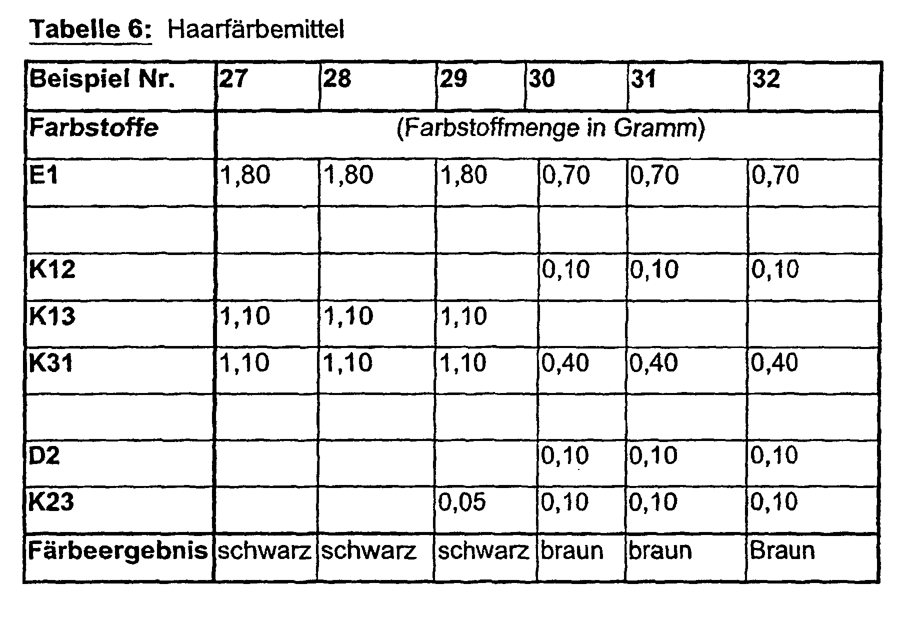

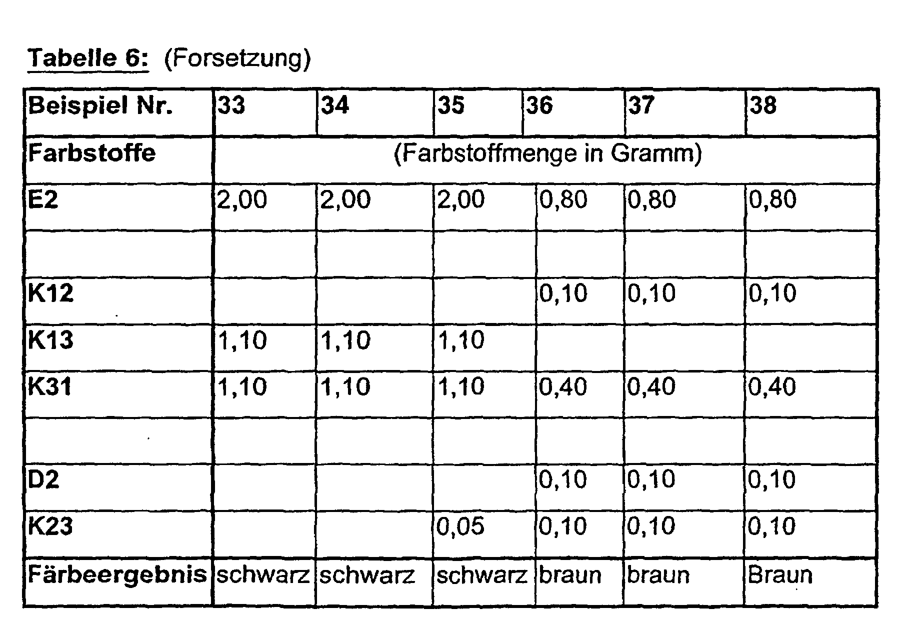

## Patentansprüche

1. 1,4-Diamino-2-alkenyl-benzol-Derivat der allgemeinen Formel (I) oder dessen physiologisch verträgliche, wasserlösliche Salze, 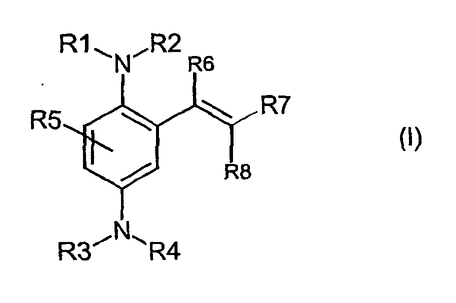 worin
**R1,R2,R3 und R4** unabhängig voneinander Wasserstoff, eine C₁-C₆-Alkylgruppe, eine C₂-C₄-Hydroxyalkylgruppe, eine C₃-C₄- Dihydroxyalkylgruppe oder eine C₁-C₄-Alkoxy-(C₁-C₂)-alkylgruppe darstellen oder R1 und R2 beziehungsweise R3 und R4 einen viergliedrigen bis achtgliedrigen aliphatischen Ring bilden, wobei mindestens 2 der Reste R1 bis R4 Wasserstoff darstellen;
**R5** gleich Wasserstoff, einem Halogenatom, einer C₁-C₄-Alkylgruppe, einer C₁-C₄ -Hydroxyalkylgruppe oder einer C₁-C₄-Alkoxygruppe ist;
**R6, R7 und R8** unabhänging voneinander gleich Wasserstoff, einem Halogenatom, einer Cyanogruppe, einer C₁-C₄ -Alkoxygruppe, einer C₁-C₆ Alkylgruppe, einer -C(O)H-Gruppe, einer -Si(CH₃)₃-Gruppe, einer C₁-C₄-Hydroxyalkylgruppe, einer C₃-C₄-Dihydroxyalkylgruppe, einer -CH2-Si(CH₃)₃-Gruppe, oder einem Rest der Formel (II) sind, 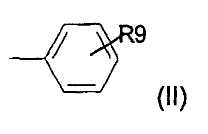 **R9** gleich Wasserstoff, einer Aminogruppe, einem Halogenatom F, Cl, Br, J), einer Nitrogruppe oder einer Hydroxygruppe ist.

2. 1,4-Diamino-2-alkenyl-benzol-Derivat nach Anspruch 1, **dadurch gekennzeichnet, dass** in der Formel (I) eine oder mehrere der Restgruppen R5 bis R8 gleich Wasserstoff sind.

3. 1,4-Diamino-2-alkenyl-benzol-Derivat nach Anspruch 1 oder 2, **dadurch gekennzeichnet, dass** in der Formel (I) die Restgruppen R1, R2, R3 und R4 gleich Wasserstoff sind.

4. 1,4-Diamino-2-alkenyl-benzol-Derivat nach einem der Ansprüche 1 bis 3, **dadurch gekennzeichnet, dass** in der Formel (I) eine oder mehrere der Restgruppen R6, R7 und R8 gleich einer C₁-C₄-Alkylgruppe oder einer C₁-C₄-Hydroxyalkylgruppe sind.

5. 1,4-Diamino-2-alkenyl-benzol-Derivat nach einem der Ansprüche 1 bis 4, **dadurch gekennzeichnet, dass** es ausgewählt ist aus 1,4-Diamino-2-styryl-benzol; 1,4-Diamino-2-propenyl-benzol; 1,4-Diamino-2-(1-methyl-propenyl)-benzol; 1,4-Diamino-2-(2-methyl-propenyl)-benzenol; 1,4-Diamino-2-(1,2-dimethyl-propenyl)-benzol; 1,4-Diamino-2-propenylbenzol; 2-(2,5-Diamino-phenyl)-prop-2-en-1-ol; 3-(2,5-Diamino-phenyl)-prop-2-en-1-ol und 3-(2,5-Diamino-phenyl)-prop-2-en-1-ol.

6. Mittel zum oxidativen Färben von Keratinfasem, auf der Basis einer Entwicklersubstanz-Kupplersubstanz-Kombination, **dadurch gekennzeichnet, dass** es als Entwicklersubstanz mindestens ein 1,4-Diamino-2-alkenyl-benzol-Derivat der Formel (I) nach einem der Ansprüche 1 bis 5 enthält.

7. Mittel nach Anspruch 6, **dadurch gekennzeichnet, daß** es das 1,4-Diamino-2-alkenyl-benzol-Derivat der Formel (I) in einer Menge von 0,005 bis 20,0 Gewichtsprozent enthält.

8. Mittel nach Anspruch 6 oder 7, **dadurch gekennzeichnet, dass** die Kupplersubstanz ausgewählt ist aus 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methylbenzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxypyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chloro-6-methyl-phenol, 3-Amino-phenol, 2-[(3-Hydroxyphenyl)-amino]-acetamid, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-lndolindion.

9. Mittel einem der Ansprüche 6 bis 8, **dadurch gekennzeichnet, dass** es zusätzlich zu dem 1,4-Diamino-2-alkenyl-benzol-Derivat der Formel (I) mindestens eine weitere Entwicklersubstanz aus der Gruppe bestehend aus 1,4-Diaminobenzol, 2,5-Diaminotoluol, 2-(2,5-Diaminophenyl)-ethylalkohol, 4-Aminophenol und seinen Derivaten, 4,5-Diaminopyrazolderivaten und Tetraaminopyrimidinen enthält.

10. Mittel nach einem der Ansprüche 6 bis 9, **dadurch gekennzeichnet, dass** die Entwicklersubstanzen und Kupplersubstanzen, bezogen auf die Gesamtmenge des Oxidationsfärbemittel, jeweils in einer Gesamtmenge von 0,005 bis 20 Gewichtsprozent enthalten sind.

11. Mittel nach einem der Ansprüche 6 bis 10 , **dadurch gekennzeichnet, daß** es zusätzlich mindestens einen direktziehenden Farbstoff enthält.

12. Mittel nach einem der Ansprüche 6 bis 11, **dadurch gekennzeichnet, daß** es einen pH-Wert von 6,5 bis 11,5 aufweist.

13. Mittel nach einem der Ansprüche 6 bis 12, **dadurch gekennzeichnet, daß** es in Form einer wässrigen oder wässrig-alkoholischen Lösung, einer Creme, eines Gels oder einer Emulsion vorliegt.

14. Mittel nach einem der Ansprüche 6 bis 13, **dadurch gekennzeichnet, dass** es ein Haarfärbemittel ist.

## Claims

1. 1,4-Diamino-2-alkenylbenzene derivative of the general formula (I) or physiologically compatible, water-soluble salts thereof, 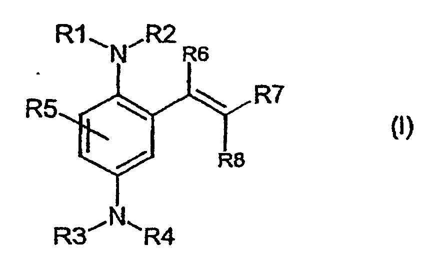 in which
**R1, R2, R3 and R4,** independently of one another, are hydrogen, a C₁-C₆-alkyl group, a C₂-C₄-hydroxyalkyl group, a C₃-C₄-dihydroxyalkyl group or a C₁-C₄-alkoxy-(C₁-C₂)-alkyl group, or R1 and R2, or R3 and R4 form a four-membered to eight-membered aliphatic ring, where at least two of the radicals R1 to R4 are hydrogen;
**R5** is hydrogen, a halogen atom, a C₁-C₉-alkyl group, a C₁-C₄-hydroxyalkyl group or a C₁-C₄-alkoxy group;
**R6, R7 and R8,** independently of one another, are hydrogen, a halogen atom, a cyano group, a C₁-C₄-alkoxy group, a C₁-C₆-alkyl group, a -C(O)H group, an -Si(CH₃)₃ group, a C₁-C₄-hydroxyalkyl group, a C₃-C₄-dihydroxyalkyl group, a -CH₂-Si(CH₃)₃ group, or a radical of the formula (II), 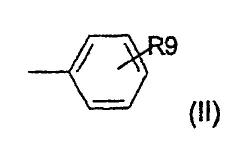 **R9** is hydrogen, an amino group, a halogen atom (F, Cl, Br, I), a nitro group or a hydroxyl group.

2. 1,4-Diamino-2-alkenylbenzene derivative according to Claim 1, **characterized in that**, in the formula (I), one or more of the radical groups R5 to R8 are hydrogen.

3. 1,4-Diamino-2-alkenylbenzene derivative according to Claim 1 or 2, **characterized in that**, in the formula (I), the radical groups R1, R2, R3 and R4 are hydrogen.

4. 1,4-Diamino-2-alkenylbenzene derivative according to one of Claims 1 to 3, **characterized in that**, in the formula (I), one or more of the radical groups R6, R7 and R8 are a C₁-C₄-alkyl group or a C₁-C₄-hydroxyalkyl group.

5. 1,4-Diamino-2-alkenylbenzene derivative according to one of Claims 1 to 4, **characterized in that** it is chosen from 1,4-diamino-2-styrylbenzene; 1,4-diamino-2-propenylbenzene; 1,4-diamino-2-(1-methylpropenyl)-benzene; 1,4-diamino-2-(2-methylpropenyl)benzene; 1,4-diamino-2-(1,2-dimethylpropenyl)benzene; 1,4-diamino-2-propenylbenzene; 2-(2,5-diaminophenyl)prop-2-en-1-ol; 3-(2,5-diaminophenyl)prop-2-en-1-ol and 3-(3,5-diamino-phenyl)prop-2-en-1-ol.

6. Agent for the oxidative dyeing of keratin fibres based on a developer substance-coupler substance combination, **characterized in that** it comprises, as developer substance, at least one 1,4-diamino-2-alkenyl-benzene derivative of the formula (I) according to any of Claims 1 to 5.

7. Agent according to Claim 6, **characterized in that** it comprises the 1,4-diamino-2-alkenylbenzene derivative of the formula (I) in an amount of from 0.005 to 20.0 percent by weight.

8. Agent according to Claim 6 or 7, **characterized in that** the coupler substance is chosen from 2,6-diamino-pyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methyl-amino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 2,4-diamino-1,5-di-(2-hydroxyethoxy)benzene, 1-(2-aminoethoxy)-2,4-diamino-benzene, 2-amino-1-(2-hydroxyethoxy)-4-methylamino-benzene, 2,4-diaminophenoxyacetic acid, 3-[di(2-hydroxyethyl)amino]aniline, 4-amino-2-di[(2-hydroxyethyl)-amino]-1-ethoxybenzene, 5-methyl-2-(1-methylethyl)-phenol, 3-[(2-hydroxyethyl)amino]aniline, 3-[(2-aminoethyl)amino]aniline, 1,3-di(2,4-diaminophenoxy)propane, di(2,4-diaminophenoxy)methane, 1,3-diamino-2,4-dimethoxy-benzene, 2,6-bis(2-hydroxyethyl)aminotoluene, 4-hydroxy-indole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]acetamide, 5-[(2-hydroxyethyl)amino]-2-methylphenol, 3-[(2-hydroxyethyl)amino]phenol, 3-[(2-methoxyethyl)amino]phenol, 5-amino-2-ethylphenol, 2-(4-amino-2-hydroxyphenoxy)ethanol, 5-[(3-hydroxypropyl)-amino]-2-methylphenol, 3-[(2,3-dihydroxypropyl)amino]-2-methylphenol, 3-[(2-hydroxyethyl)amino]-2-methylphenol, 2-amino-3-hydroxypyridine, 5-amino-4-chloro-2-methylphenol, 1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxole, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

9. Agent according to one of Claims 6 to 8, **characterized in that**, in addition to the 1,4-diamino-2-alkenylbenzene derivative of the formula (I), it comprises at least one further developer substance from the group consisting of 1,4-diaminobenzene, 2,5-diaminotoluene, 2-(2,5-diaminophenyl)ethyl alcohol, 4-aminophenol and its derivatives, 4,5-diaminopyrazole derivatives and tetraaminopyrimidines.

10. Agent according to one of Claims 6 to 9, **characterized in that** the developer substances and coupler substances are in each case present in a total amount of from 0.005 to 20 percent by weight, based on the total amount of the oxidation colorant.

11. Agent according to one of Claims 6 to 10, **characterized in that** it additionally comprises at least one direct dye.

12. Agent according to one of Claims 6 to 11, **characterized in that** it has a pH of from 6.5 to 11.5.

13. Agent according to one of Claims 6 to 12, **characterized in that** it is in the form of an aqueous or aqueous-alcoholic solution, a cream, a gel or an emulsion.

14. Agent according to one of Claims 6 to 13, **characterized in that** it is a hair colorant.

## Revendications

1. Dérivé de 1,4-diamino-2-alcénylbenzène de formule générale (I) ou sels hydrosolubles, physiologiquement acceptables, d'un tel dérivé, 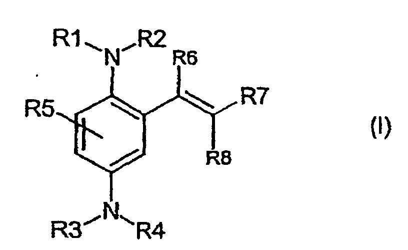 formule dans laquelle
**R1, R2, R3** et **R4** représentent, indépendamment les uns des autres, un atome d'hydrogène, un groupe alkyle en C₁-C₆, un groupe hydroxyalkyle en C₂-C₄, un groupe dihydroxyalkyle en C₃-C₄ ou un groupe alcoxy(C₁-C₄)-alkyle(C₁-C₂) ou bien **R1** et **R2** ou, respectivement, **R3** et **R4,** forment un cycle aliphatique à 4 chaînons ou à 8 chaînons, au moins deux des radicaux R1 à R4 représentant un atome d'hydrogène ;
**R5** représente un atome d'hydrogène ou d'halogène, un groupe alkyle en C₁-C₄, un groupe hydroxyalkyle en C₁-C₄ ou un groupe alcoxy en C₁-C₄ ;
**R6, R7** et **R8** représentent, indépendamment les uns des autres, un atome d'hydrogène ou d'halogène ou un groupe cyano, un groupe alcoxy en C₁-C₄, un groupe alkyle en C₁-C₆, un groupe -C(O)H, un groupe -Si(CH₃)₃, un groupe hydroxyalkyle en C₁-C₄, un groupe dihydroxyalkyle en C₃-C₄, un groupe -CH₂-Si(CH₃)₃ ou un radical de formule (II), 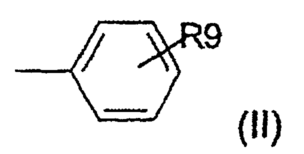 **R9** est un atome d'hydrogène, un groupe amino, un atome d'halogène (F, Cl, Br, I), un groupe nitro ou un groupe hydroxy.

2. Dérivé de 1,4-diamino-2-alcénylbenzène selon la revendication 1, **caractérisé en ce que**, dans la formule (I), un ou plusieurs des groupes R5 à R8 représentent un atome d'hydrogène.

3. Dérivé de 1,4-diamino-2-alcénylbenzène selon la revendication 1 ou 2, **caractérisé en ce que**, dans la formule (I), les groupes R1, R2, R3 et R4 sont des atomes d'hydrogène.

4. Dérivé de 1,4-diamino-2-alcénylbenzène selon l'une quelconque des revendications 1 à 3, **caractérisé en ce que**, dans la formule (I), un ou plusieurs des groupes R6, R7 et R8 représentent un groupe alkyle en C₁-C₄ ou un groupe hydroxyalkyle en C₁-C₄.

5. Dérivé de 1,4-diamino-2-alcénylbenzène selon l'une quelconque des revendications 1 à 4, **caractérisé en ce qu'**il est choisi parmi le 1,4-diamino-2-styrylbenzène, le 1,4-diamino-2-propénylbenzène, le 1,4-diamino-2-(1-méthylpropényl)benzène, le 1,4-diamino-2-(2-méthylpropényl)benzénol, le 1,4-diamino-2-(1,2-diméthylpropényl)benzène, le 1,4-diamino-2-propénylbenzène, le 2-(2,5-diaminophényl)prop-2-én-1-ol, le 3-(2,5-diaminophényl)prop-2-én-1-ol et le 3-(2,5-diaminophényl)prop-2-én-1-ol.

6. Composition pour la teinture par oxydation de fibres de kératine, à base d'une association développeur-coupleur, **caractérisée en ce qu'**elle contient en tant que développeur au moins un dérivé de 1,4-diamino-2-alcénylbenzène de formule (I) selon l'une quelconque des revendications 1 à 5.

7. Composition selon la revendication 6, **caractérisée en ce qu'**elle contient le dérivé de 1,4-diamino-2-alcénylbenzène de formule (I) en une quantité de 0,005 à 20 % en poids.

8. Composition selon la revendication 6 ou 7, **caractérisée en ce que** le coupleur est choisi parmi la 2,6-diaminopyridine, le 2-amino-4-[(2-hydroxyéthyl)-amino]anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxypyridine, la 3-amino-6-méthoxy-2-(méthylamino)pyridine, la 2,6-diamino-3,5-diméthoxypyridine, la 3,5-diamino-2,6-diméthoxypyridine, le 1,3-diaminobenzène, le 2,4-diamino-1-(2-hydroxyéthoxy)-benzène, le 2,4-diamino-1,5-di(2-hydroxyéthoxy)benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)amino]-aniline, le 4-amino-2-di[(2-hydroxyéthyl)amino]-1-éthoxybenzène, le 5-méthyl-2-(1-méthyléthyl)phénol, la 3-[(2-hydroxyéthyl)amino]aniline, la 3-[(2-aminoéthyl)-amino]aniline, le 1,3-di(2,4-diaminophénoxy)propane, le di(2,4-diaminophénoxy)méthane, le 1,3-diamino-2,4-diméthoxybenzène, le 2,6-bis(2-hydroxyéthyl)aminotoluène, le 4-hydroxy-indole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxyphényl)amino]acétamide, le 5-[(2-hydroxyéthyl)amino]-2-méthylphénol, le 3-[(2-hydroxyéthyl)amino]phénol, le 3-[(2-méthoxyéthyl)amino]phénol, le 5-amino-2-éthylphénol, le 2-(4-amino-2-hydroxyphénoxy)éthanol, le 5-[(3-hydroxypropyl)amino]-2-méthylphénol, le 3-[(2,3-dihydroxypropyl)amino]-2-méthylphénol, le 3-[(2-hydroxyéthyl)amino]-2-méthylphénol, la 2-amino-3-hydroxypyridine, le 5-amino-4-chloro-2-méthylphénol, le 1-naphtol, le 1,5-dihydroxynaphtalène, le 1,7-dihydroxynaphtalène, le 2,3-dihydroxynaphtalène, le 2,7-dihydroxynaphtalène, l'acétate de 2-méthyl-1-naphtol, le 1,3-dihydroxybenzène, le 1-chloro-2,4-dihydroxybenzène, le 2-chloro-1,3-dihydroxybenzène, le 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxyphénol, la 3,4-méthylènedioxyaniline, le 5-[(2-hydroxyéthyl)amino]-1,3-benzodioxole, le 6-bromo-1-hydroxy-3,4-méthylènedioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxy-indole, la 5,6-dihydroxyindoline, le 5-hydroxy-indole, le 6-hydroxy-indole, le 7-hydroxy-indole et la 2,3-indolinedione.

9. Composition selon l'une quelconque des revendications 6 à 8, **caractérisée en ce que**, en plus du dérivé de 1,4-diamino-2-alcénylbenzène de formule (I), elle contient au moins un autre développeur choisi dans le groupe constitué par le 1,4-diaminobenzène, le 2,5-diaminotoluène, l'alcool 2-(2,5-diaminophényl)-éthylique, le 4-aminophénol et ses dérivés, des dérivés de 4,5-diaminopyrazole et les tétraaminopyrimidines.

10. Composition selon l'une quelconque des revendications 6 à 9, **caractérisée en ce que** les développeurs et les coupleurs sont contenus, respectivement, en une quantité totale de 0,005 à 20 % en poids, par rapport à la quantité totale de la composition de teinture par oxydation.

11. Composition selon l'une quelconque des revendications 6 à 10, **caractérisée en ce qu'**en outre elle contient au moins un colorant direct.

12. Composition selon l'une quelconque des revendications 6 à 11, **caractérisée en ce qu'**elle présente un pH de 6,5 à 11,5.

13. Composition selon l'une quelconque des revendications 6 à 12, **caractérisée en ce qu'**elle se trouve sous forme d'une solution aqueuse ou aqueuse-alcoolique, d'une crème, d'un gel ou d'une émulsion.

14. Composition selon l'une quelconque des revendications 6 à 13, **caractérisée en ce qu'**il s'agit d'une composition de teinture pour cheveux.
